(19) Europäisches Patentamt
European Patent Office
Office européen des brevets

(11) Veröffentlichungsnummer: **0 181 529 B1**

(12) **EUROPÄISCHE PATENTSCHRIFT**

(45) Veröffentlichungstag der Patentschrift: **27.03.91**

(51) Int. Cl.⁵: **C07D 249/08**, C07B 53/00, C07B 57/00, A61K 31/41, C07D 233/60, C07D 233/61, C07D 403/08, //C07C309/63

(21) Anmeldenummer: **85113314.0**

(22) Anmeldetag: **21.10.85**

Die Akte enthält technische Angaben, die nach dem Eingang der Anmeldung eingereicht wurden und die nicht in dieser Patentschrift enthalten sind.

# (54) Optisch aktives 2-(4-Chlorphenoxymethyl)-3,3-dimethyl-1-(1,2,4-triazol-1-yl)-2-butanol, ein Verfahren zu dessen Herstellung und dessen Verwendung als Antimykotikum.

(30) Priorität: **02.11.84 DE 3440118**
**02.11.84 DE 3440112**

(43) Veröffentlichungstag der Anmeldung:
**21.05.86 Patentblatt 86/21**

(45) Bekanntmachung des Hinweises auf die Patenterteilung:
**27.03.91 Patentblatt 91/13**

(84) Benannte Vertragsstaaten:
**AT BE CH DE FR GB IT LI NL SE**

(56) Entgegenhaltungen:
**EP-A- 0 040 345**
**EP-A- 0 054 431**
**EP-A- 0 098 942**
**EP-A- 0 043 419**
**EP-A- 0 089 922**
**US-A- 4 036 970**

(73) Patentinhaber: **BAYER AG**

**W-5090 Leverkusen 1 Bayerwerk(DE)**

(72) Erfinder: **Kraatz, Udo, Dr.**
**Andreasstr. 22a**
**W-5090 Leverkusen(DE)**
Erfinder: **Holmwood, Graham, Dr.**
**Krutscheider Weg 105**
**W-5600 Wuppertal 1(DE)**
Erfinder: **Berg, Dieter, Dr.**
**Gellertweg 27**
**W-5600 Wuppertal 1(DE)**
Erfinder: **Plempel, Manfred, Dr.**
**Zwengenberger Str. 3c**
**W-5657 Haan(DE)**

**EP 0 181 529 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein neues optisch aktives Azolylcarbinol-Derivat, ein Verfahren zu dessen Herstellung aus einem bekannten, racemischem Oxiran und dessen Verwendung als Antimykotikum.

Es ist bereits bekannt geworden, daß man bestimmte racemische Azol-Derivate auf dem klassischen Weg in die optischen Antipoden spalten kann, indem man sie in einer ersten Stufe mit optisch aktiven Säuren, gegebenenfalls in Gegenwart eines Verdünnungsmittels, umsetzt; dann in einer zweiten Stufe die entsprechenden Salze aufgrund ihrer unterschiedlichen Löslichkeit trennt, und danach in einer dritten Stufe die optischen Antipoden aus den entsprechenden Salzen mit Hilfe von Basen, gegebenenfalls in Gegenwart eines Verdünnungsmittels, in Freiheit setzt (vgl. z.B. EP-A 0 004 918 und DE-A 3 302 122). Dieses Verfahren ist jedoch nicht auf alle Azol-Derivate anwendbar. Wie eigene Versuche zeigten, ist es nicht möglich, kristalline Salze von β-Hydroxyethyl-azolyl-Derivaten vom Typ der Verbindung der Formel (I) mit optisch aktiven Säuren herzustellen.

Außerdem ist bekannt, daß Epoxide mit Säuren geöffnet werden können (vgl. Houben-Weyl, Methoden der organischen Chemie, Band VI/3, S. 448 ff (1965)). Auch dieses Verfahren ist jedoch nicht auf alle Epoxide übertragbar. Wie eigene Versuche zeigten, ist es nicht möglich, mit optisch aktiven Säuren, wie beispielsweise Mandelsäure oder Weinsäure, Epoxide vom Typ der Verbindung der Formel (II) zu öffnen. Die gewünschte Reaktion konnte erst durch Zusatz von Bortrifluoridetherat zur Reaktionslösung erreicht werden.

Ferner ist aus der EP-A 0 098 942 bekannt, daß optisch aktive 3-Alkylthio-2-dichlorphenyl-1-imidazol-1-yl-2-propanole der Formel

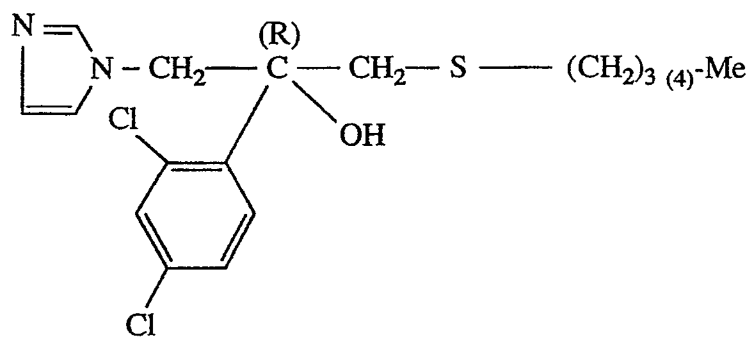

erhalten werden können, wenn man in einer ersten Stufe die entsprechenden Epoxide hydrolisiert; in einer zweiten Stufe die entsprechenden Diole mit optisch aktiven Säuren, wie z.B. Milchsäure, verestert; in einer dritten Stufe die diastereomeren Ester trennt und hydrolisiert; in einer vierten Stufe das entsprechende Sulfonat herstellt; und in einer fünften Stufe mit Alkylmercaptan umsetzt. Dieses Verfahren hat den Nachteil einer Mehrstufenreaktion.

Es ist auch bereits bekannt geworden, daß racemisches 2-(4-Chlorphenoxymethyl)-3,3-dimethyl-1-(1,2,4-triazol-1-yl)-2-butanol gute antimykotische Eigenschaften aufweist (vgl. EP-A 0 043 419). Die pharmakologischen Eigenschaften dieser Verbindung sind jedoch für die isolierten Enantiomeren unterschiedlich.

Schließlich sind aus der US-A 4 036 970 bestimmte Imidazol-Derivate bekannt, die zur Bekämpfung von humanpathogenen Pilzen eingesetzt werden können. Es werden jedoch keine Verbindungen beschrieben, in denen das Kohlenstoffatom in 2-Stellung zum Imidazol-Rest nur Substituenten trägt, die von Wasserstoff verschieden sind. Außerdem wird keine optisch aktive Verbindung speziell offenbart.

Es wurde nun das (-)-Enantiomere von 2-(4-Chlorphenoxymethyl)-3,3-dimethyl-1-(1,2,4-triazol-1-yl)-2-butanol der Formel

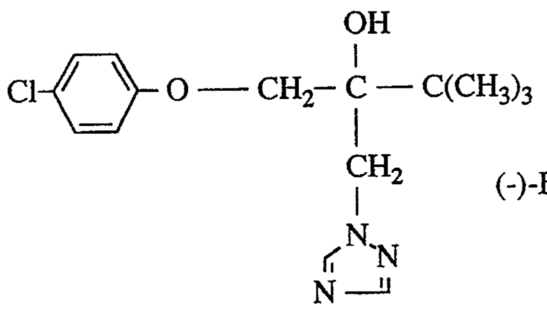

(-)-Form (I)

gefunden.

Weiterhin wurde gefunden, daß man das (-)-Enantiomere von 2-(4-Chlorphenoxymethyl)-3,3-dimethyl-1-(1,2,4-triazol-1-yl)-2-butanol erhält, wenn man in einer 1. Stufe racemisches 2-(4-Chlorphenoxymethyl)-2-tert.-butyl-oxiran der Formel

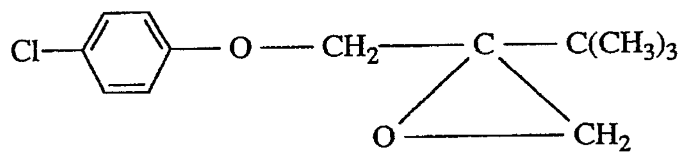

(II)

mit starken, optisch aktiven Säuren gegebenenfalls in Gegenwart eines Verdünnungsmittels umsetzt, das entstehende diastereomere Estergemisch in die reinen diastereomeren Komponenten auftrennt und in einer 2. Stufe mit 1,2,4-Triazol in Gegenwart eines Verdünnungsmittels und gegebenenfalls in Gegenwart einer Base umsetzt.

Überraschenderweise zeigt das (-)-Enantiomere von 2-(4-Chlorphenoxymethyl)-3,3-dimethyl-1-(1,2,4-triazol-1-yl)-2-butanol der Formel (I) bei sehr guter allgemeiner antimykotischer Wirksamkeit insbesondere eine höhere Sterolsynthese-Hemmung und somit bessere pharmakologische Eigenschaften als das bekannte entsprechende Racemat. Der erfindungsgemäße Stoff stellt somit eine wertvolle Bereicherung der Pharmazie dar.

Es ist auch als äußerst überraschend zu bezeichnen, daß sich das optisch aktive Azolylcarbinol-Derivat der Formel (I) nach dem erfindungsgemäßen Verfahren in hoher Ausbeute herstellen läßt. Aufgrund des Standes der Technik war zu erwarten, daß ohne Katalysator, wie z.B. Bortrifluoridetherat, die gewünschte Ringöffnung nicht eintritt.

Das erfindungsgemäße Verfahren besitzt den Vorteil, die optischen Antipoden des Azolyl-Derivates der Formel (I) in hoher Ausbeute zu erhalten, bei dem die klassische Methode der Racematspaltung nicht zum Ziel führt.

Verwendet man neben dem 2-(4-Chlorphenoxymethyl)-2-tert.-butyl-oxiran als Ausgangsstoff die d(+)-Campher-10-sulfonsäure als optisch aktive Säure, so kann der Reaktionsablauf durch das folgende Formelschema wiedergegeben werden:

1. Stufe

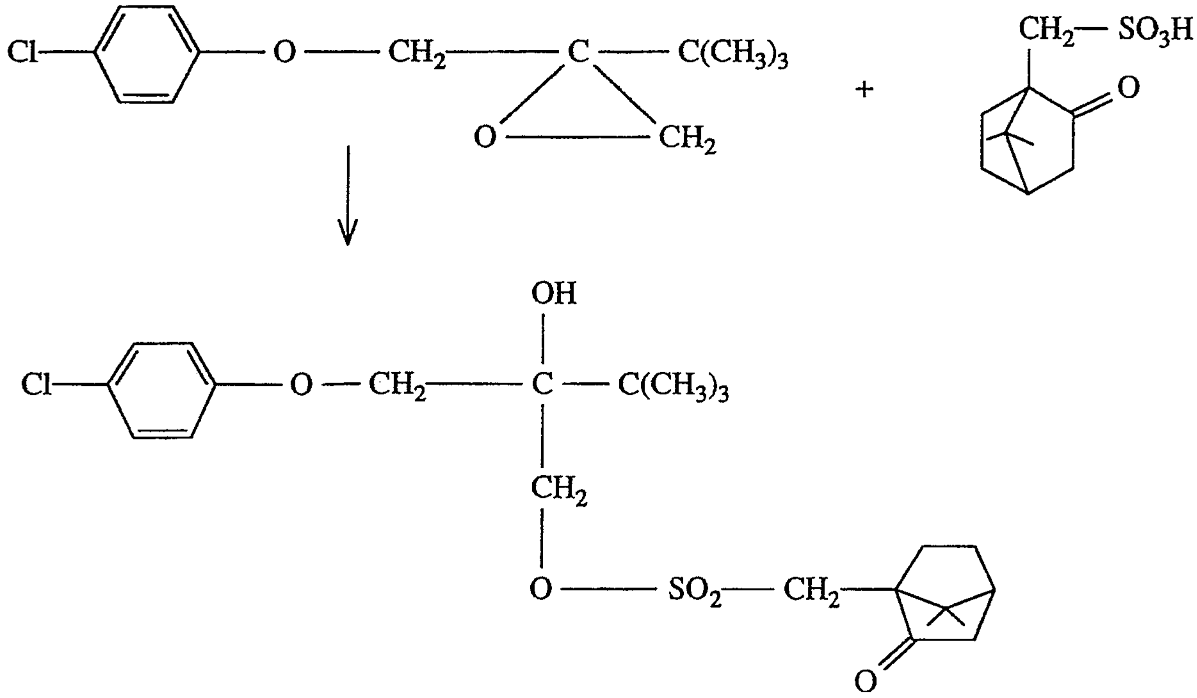

[1(-)Carbinol/d(+)-Säure sowie d(+)Carbinol/d(+)-Säure]

Trennung

[1(-)Carbinol/d(+)-Säure]

[d(+)Carbinol/d(+)-Säure]

2. Stufe

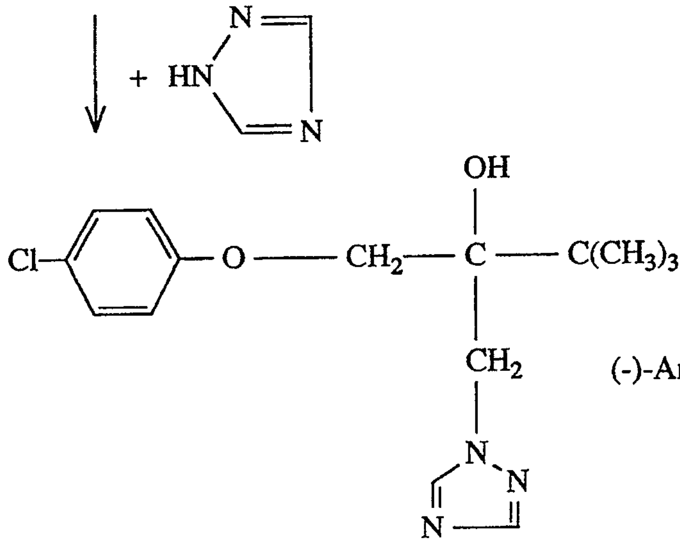

(-)-Antipode

Das als Ausgangsstoff zu verwendende racemische 2-(4-Chlorphenoxymethyl)-2-tert.-butyl-oxiran der Formel ist bekannt (vgl. z.B. EP-A 0 040 345).

Die dem optisch aktiven Azolylcarbinol-Derivat der Formel (I) entsprechende racemische Verbindung ist ebenfalls bekannt.

Bei der Durchführung des erfindungsgemäßen Verfahrens wird das Oxiran der Formel (II) in der 1. Stufe mit optisch aktiven Sulfonsäuren umgesetzt. Hierzu gehören vorzugsweise Campher-10-sulfonsäure, 3-Brom-10-camphersulfonsäure und 3-Brom-8-camphersulfonsäure.

In bestimmten Fällen können auch andere starke, optisch aktive Säuren eingesetzt werden. Hierbei seien insbesondere optisch aktive Phosphorsäuren genannt, wie beispielsweise 1,1'-Binaphthyl-2,2'-diyl-phosphat der Formel

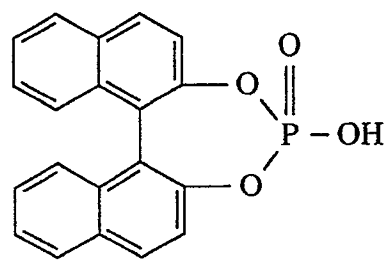

Als Verdünnungsmittel kommen bei der Durchführung der ersten Stufe des erfindungsgemäßen Verfahrens inerte organische Lösungsmittel in Frage. Hierzu gehören vorzugsweise Nitrile, wie Acetonitril; Keton, wie Methylethylketon oder Aceton; Ether, wie Tetrahydrofuran oder Dioxan; und halogenierte Kohlenwasserstoffe, wie Chloroform oder Methylenchlorid.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 0 und 100° C, vorzugsweise zwischen 10 und 60° C.

Bei der Durchführung der ersten Stufe des erfindungsgemäßen Verfahrens arbeitet man vorzugsweise in äquimolaren Mengen. Die Trennung der beiden diastereomeren Verbindungen erfolgt in üblicher Art und Weise aufgrund unterschiedlicher physikochemischer Eigenschaften, wie beispielsweise durch fraktionierte Kristallisation oder chromatographische Trennmethoden.

Als Verdünnungsmittel kommen bei der Durchführung der zweiten Stufe des erfidungsgemäßen Verfahrens ebenfalls inerte organische Lösungsmittel in Frage. Hierzu gehören vorzugsweise Nitrile, wie Acetonitril; Alkohole, wie Ethanol oder Propanol; Ketone, wie Methylethylketon oder Aceton; Ester, wie Essigester; Ether, wie Tetrahydrofuran oder Dioxan; und Amide, wie Dimethylformamid.

Als Basen kommen bei der Durchführung der zweiten Stufe des erfindungsgemäßen Verfahrens alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid;

Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 0 und 150° C, vorzugsweise zwischen 40 und 120° C.

Bei der Durchführung der zweiten Stufe des erlindungsgemäßen Verfahrens setzt man vorzugsweise auf 1 Mol Ester 1 bis 4 Mol Azol und gegebenenfalls 1 bis 2 Mol Base ein. Die Isolierung des Endproduktes erfolgt in allgemein üblicher Weise.

Das nach dem erfindungsgemäßen Verfahren herstellbare optisch aktive Azolylcarbinol-Derivat zeigt hervorragende biologische Wirkungen, wobei der einzelne Antipode eine bessere Wirksamkeit als das entsprechende Racemat hat.

Die erfindungsgemäße Verbindung der Formel (I) weist antimikrobielle, insbesondere starke antimykotische Wirkungen auf. Sie besitzt ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Microsporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören phamarzeutische Zubereitungen, die neben nichttoxischen, inerten pharmazeutisch geeigneten Trägerstoffen den erfindungsgemäßen Wirkstoff enthalten oder die aus dem erfindungsgemäßen Wirkstoff bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder $\frac{1}{2}$, $\frac{1}{3}$ oder $\frac{1}{4}$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tableten, Dragees, Kapseln, Pillen und Granulate können den Wirkstoff neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonstearat, (h) Absorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulaten können mit den üblichen gegebenenfalls Opakisierungsmittel enthaltenden Überzügen und Hüllen versehen sein und so zusammengesetzt sein, daß sie den Wirkstoff nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der Wirkstoff kann gegebenenfalls mit einem oder mehreren oder oben angegebenen Trägerstoffe auch in mikroverkapselter Form vorliegen.

Suppositorien können neben dem Wirkstoff die üblichen wasserlöslichen wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem Wirkstoff die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem Wirkstoff die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem Wirkstoff die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem Wirkstoff die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksame Verbindung soll in den oben aufgeführten pharmazeutischen Zubereitungen vorzugsweise in einer Konzentration von etwa 0,1 bis 99,5, vorzugsweise von 0,5 bis 95, Gew.-% der Gesamtmischung vorhanden sein.

Die oben aufgeführten pharmazeutischen Zubereitungen können außer dem erfindungsgemäßen Wirkstoff auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen der Wirkstoffes mit dem oder den Trägerstoffen.

Zur vorliegenden Erfindung gehört auch die Verwendung des erfindungsgemäßen Wirkstoffs zur Herstellung von pharmazeutischen Zubereitungen, die den erfindungsgemäßen Wirkstoff enthalten, für die Human- und Veterinärmedizin zur Verhütung, Besserung und/oder Heilung der oben aufgeführten Erkrankungen.

Der Wirkstoff oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den erfindungsgemäßen Wirkstoff in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation wird der erfindungsgemäße Wirkstoff in Gesamtmengen von etwa 2,5 bis etwa 200, vorzugsweise 5 bis 150, mg/kg Körpergewicht je 24 Stunden und bei parenteraler Applikation in Gesamtmengen von etwa 2,5 bis etwa 50, vorzugsweise 1 bis 25, mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

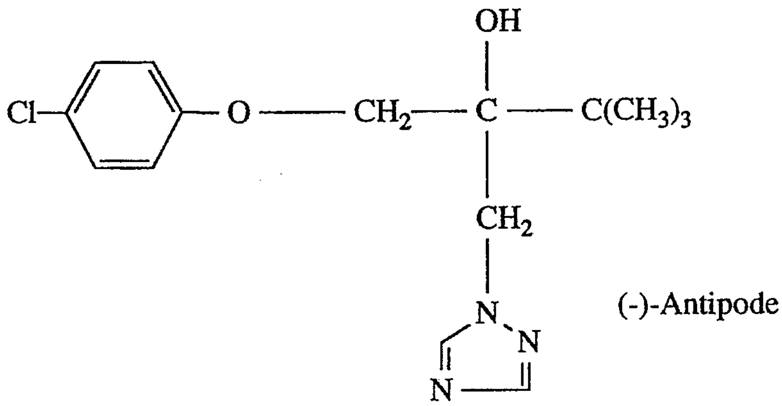

(-)-Antipode

1. Stufe

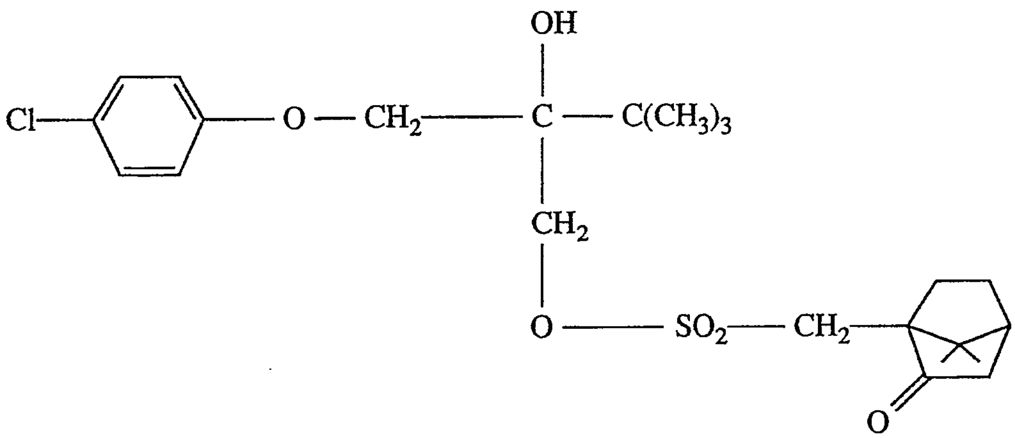

Zu 12 g (0,05 Mol) racemischen 2-(4-Chlorphenoxymethyl)-2-tert.-butyl-oxiran in 150 ml Acetonitril werden bei 20 °C unter Rühren 12,5 g (0,05 Mol) d(+)-Campher-10-sulfonsäure gegeben. Man läßt über Nacht bei Raumtemperatur stehen, gießt in Wasser und extrahiert mit Methylenchlorid. Die organische Phase wird zweimal mit Wasser gewaschen, über Magnesiumsulfat getrocknet und im Vakuum eingeengt. Man erhält 18 g an Diastereomerengemisch des d(+)-Campher-10-sulfonsäure-[2-(4-chlorphenoxymethyl)-3,3-dimethyl-2-hydroxy-1-butyl]-esters als viskoses Öl, aus dem teilweise ein reines Diasteromer ($F_p$:103 °C) auskristallisiert.

Mittels HPLC an Kieselgel im System Hexan/Isopropylether wird das Diastereomerengemisch getrennt. Man erhält

a) 5,2 g Fraktion 1 als farbloses Öl mit einem Drehwinkel von $[\alpha]_D^{20}$ = + 21,6° ($CHCl_3$, C = 0,67) und

b) 5,0 g Fraktion 2 vom Schmelzpunkt 103 °C mit einem Drehwinkel von $[\alpha]_D^{20}$ = + 32.8° ($CHCl_3$, C = 1,02).

2. Stufe

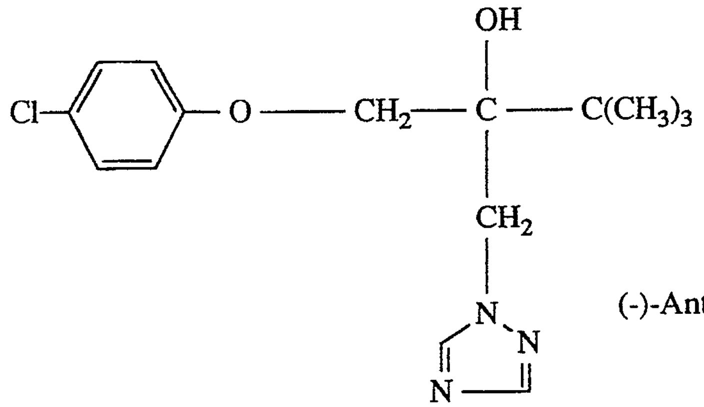

(-)-Antipode

5,2 g (11 mMol) d(+)Campher-l0-sulfonsäure-[2-(4-chlorphenoxymethyl)-3,3-dimethyl-2-hydroxy-1-butyl]-ester gemäß Fraktion 1 der ersten Stufe werden mit 3 g (43 mMol) 1,2,4-Triazol und 3 g (21 mMol) Kaliumcarbonat in 60 ml Acetonitril unter Rühren 8 Stunden unter Rückfluß erhitzt. Anschließend gibt man das Reaktionsgemisch auf Wasser, extrahiert mit Methylenchlorid und engt die organische Phase ein. Das Rohprodukt wird mittels Säulenchromatographie im System Chloroform/Essigester (3:1) gereinigt. Man erhält 2,2 g (68 % der Theorie) vom (-)-Antipoden des 2-(4-Chlorphenoxymethyl)-3,3-dimethyl-1-(1,2,4-triazol-1-yl)-2-butanols vom Schmelzpunkt 57 °C mit einem Drehwinkel von $[\alpha]_D^{20}$ = 111,4° ($CHCl_3$).

Beispiel 2

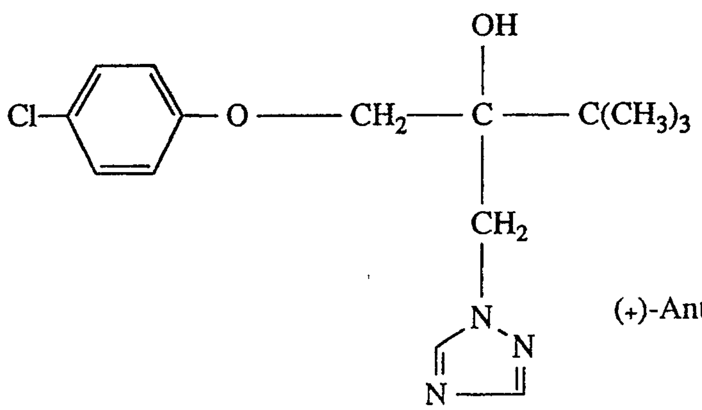

(+)-Antipode (nicht beansprucht)

1. Stufe

Vergleich hierzu Beispiel 1,1. Stufe, Fraktion 2.

2. Stufe

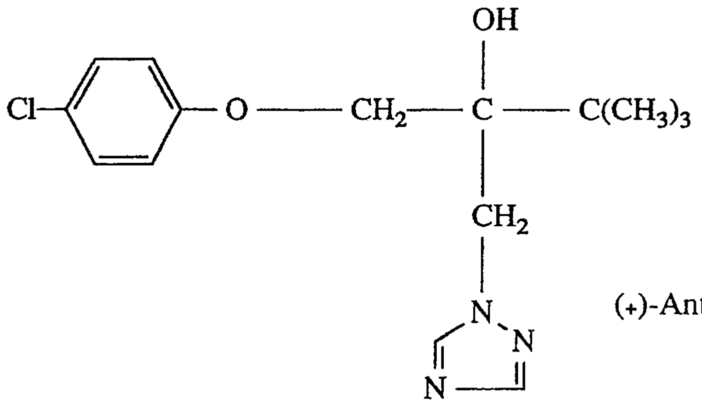

(+)-Antipode

8,0 g (17 mMol) d(+)-Campher-10-sulfonsäure-[2-(4-chlorphenoxymethyl)-3,3-dimethyl-2-hydroxy-1-butyl]-ester gemäß Fraktion 2 der 1. Stufe des Beispiels 1 werden mit 5 g (72 mMol) 1,2,4-Triazol und 5 g (35 mMol) Kaliumcarbonat in 80 ml Acetonitril unter Rühren 8 Stunden unter Rückfluß erhitzt. Anschließend gibt man das Reaktionsgemisch auf Wasser, extrahiert mit Methylenchlorid und engt die organische Phase ein. Der ölige Rückstand wird in Cyclohexan gelöst. Hierbei kristallisiert das symmetrische Triazolderivat aus (1,2 g vom Schmelzpunkt 220° C), welches abfiltriert wird.

Das Filtrat wird im Vakuum eingeengt und der ölige Rückstand wird mit Petrolether zur Kristallisation gebracht. Man erhält 3,2 g (57 % der Theorie) vom (+)-Antipode des 2-(4-Chlorphenoxymethyl)-3,3-dimethyl-1-(1,2,4-triazol-1-yl)-2-butanols vom Schmelzpunkt 57° C mit einem Drehwinkel von $[\alpha]_D^{20}$ = + 113° C ($CHCl_3$).

Verwendungsbeispiel

Beispiel A / Sterolsynthese-Hemmung durch Candida albicans

Versuchsbeschreibung :

Anzucht der Keime und Inkubation mit den Wirkstoffen:

250 ml Enghals-Erlenmeyerkolben wurden mit 95 ml Nährlösung nach Kimmig gefüllt und mit dichtsitzenden Wattestopfen verschlossen. Kimmig-Medium hat folgende Zusammensetzung:

| | |
|---|---|
| Nährbrühe (Difco 003) | 13 g |
| Glycerin p.a. | 5 g |
| Bacto-Pepton (Difco 0118) | 8,6 g |
| Glucose | 10 g |
| NaCl p.a. | 9 g |
| Entmineralisiertes Wasser ad | 1.000 g |

Nach dem Sterilisieren (15 Minuten hei 120° C) wurden die Kolben beimpft. Zur Beimpfung dienten: 48-Stunden-Kulturen auf Kimmig-Schrägröhrchen. Zum Abschwemmen wurde sterile physiologische NaCl-Lösung verwendet, und die Kulturoberflächen wurden mit einem Glasspatel abgerieben. Anschließend wurden die Keimsuspensionen, über sterile Gazefilter filtriert. Für jeden Kolben wurden aus den Filtraten photometrischer Keiminokula von 5 x $10^7$ Keimen pro 5 ml Kimmig-Medium hergestellt und diese den Kolben zugesetzt. Die Zahl wahrscheinlich keimfähiger Partikel pro ml Nährsubstrat in den Kolben betrug damit ca. 5 x $10^5$/ml.

Nach Beimpfung wurden die Kolben in einem Schüttelschrank (Clim-O-shake, A. Kühner, Basel) bei 28° C und einer Schüttelfrequenz von 95 UpM bebrütet. Die Wirkstoffe - gelöst in jeweils 1 ml absolutem

Ethanol - wurden unmittelbar vor der Bebrütung zugesetzt. Bei den wirkstofffreien Kontrollkolben wurde nur 1 ml Ethanol zugegeben.

Die Bebrütung der Candida-Kulturen dauerte 48 Stunden. Nach der Bebrütung wurden die bewachsenen Kulturkolben in Zentrifugengläser entleert und diese 15 Minuten bei 5.000 UpM zentrifugiert. Nach Waschen des Zentrifugates mit physiologischer NaCl-Lösung und erneutem Zentrifugieren wurden die sedimentierten Keime zur weiteren Verarbeitung in einem Gemisch aus Chloroform und Methanol (2:1) aufgenommen.

Extraktion mit Isolierung der Sterole erfolgte nach Ann. hytopathol. 10 , 1980, 45.

Die quantitative Analyse wurde gaschromatographisch entsprechend Phytochem. 18, 1979, 445 durchgeführt.

## Ergebnis

| Wirkstoff | Konzentr. in ppm | Gesamtsterolmenge in Flächeneinheiten | Gesamtsterolmenge in γ/20 ml Testansatz |
|---|---|---|---|
| 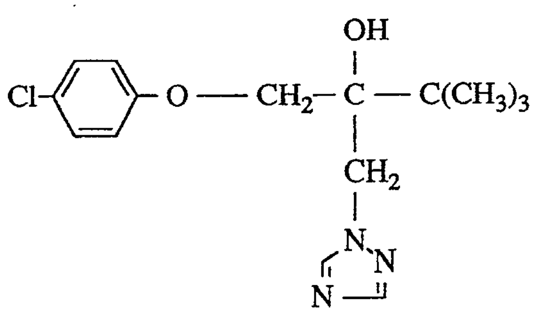 | | | |
| | 1 | kein Wachstum beobachtbar | |
| (-)-Antipode | 0,1 | 29,4 | 97,0 |
| (+)-Antipode | 1 | 101,3 | 334,3 |
| (±)-Racemat | 1 | 41,0 | 135,3 |
| Kontrolle | - | 164,4 | 542,5 |

Diese Ergebnisse zeigen die bessere Sterolsynthese-Hemmung der erfindungsgemäßen (-)-Antipode im Vergleich mit der entsprechenden (+)-Antipode und dem Racemat, was zu besseren pharmakologischen Eigenschaften der (-)-Antipode führt.

### Ansprüche

1. (-)-Enantiomer von 2-(4-Chlorphenoxymethyl)-3,3-dimethyl-1-(1,2,4-triazol-1-yl)-2-butanol der Formel

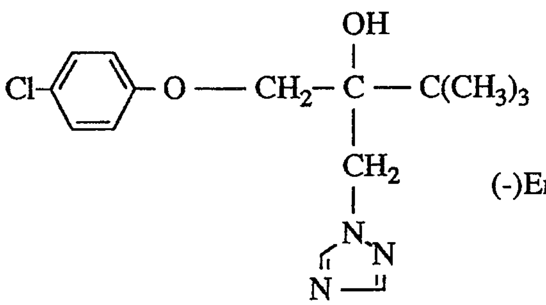

(-)Enantiomer (I)

2. Verfahren zur Herstellung des (-)-Enantiomeren von 2-(4-Chlorphenoxymethyl)-3,3-dimethyl-1-(1,2,4-triazol-1-yl)-2-butanol der Formel

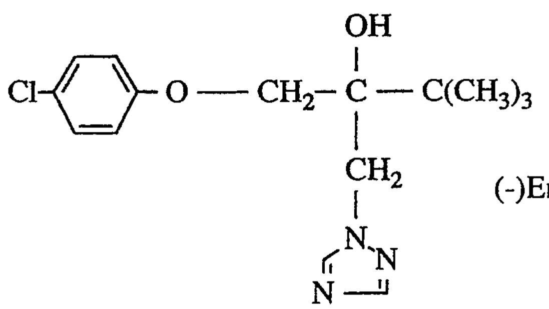

(-)Enantiomer (I)

dadurch gekennzeichnet, daß man in einer 1. Stufe racemisches 2-(4-Chlorphenoxymethyl)-2-tert.-butyl-oxiran der Formel

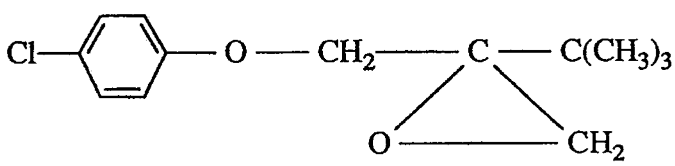

(II)

mit starken, optisch aktiven Säuren gegebenenfalls in Gegenwart eines Verdünnungsmittels umsetzt, das entstehende diastereomere Estergemisch in die reinen diastereomeren Komponenten auftrennt und in einer 2. Stufe mit 1,2,4-Triazol in Gegenwart eines Verdünnungsmittels und gegebenenfalls in Gegenwart einer Base umsetzt.

3. Verfahren gemäß Anspruch 2, dadurch gekennzeichnet daß man Campher-10-sulfonsäure, 3-Brom-10-camphersulfonsäure, 3-Brom-8-camphersulfonsäure oder 1,1'-Binaphthyl-2,2-diyl-phosphat als starke optisch aktive Säuren einsetzt.

4. Verfahren gemäß Anspruch 2, dadurch gekennzeichnet, daß man beim ersten Reaktionsschritt im Temperaturbereich von 0 bis 100 °C, beim zweiten im Temperaturbereich von 0 bis 150 °C arbeitet.

5. Verfahren gemäß Anspruch 2, dadurch gekennzeichnet, daß man im ersten Reaktionsschritt äquimolare Mengen der Komponenten, im zweiten Reaktionsschritt pro Mol Ester mit 1 bis 4 Mol Triazol und gegebenenfalls 1 bis 2 Mol Base einsetzt.

6. Verwendung des (-)-Enantiomeren von 2-(4-Chlorphenoxymethyl)-3,3-dimethyl-1-(1,2,4-triazol-1-yl)-2-butanol der Formel (I) gemäß Anspruch 1 zur Herstellung von antimykotischen Mitteln.

## Claims

1. (-)-Enantiomer of 2-(4-ohlorophenoxymethyl)-3,3-dimethyl-1-(1,2,4-triazol-1-yl)-2-butanol of the formula

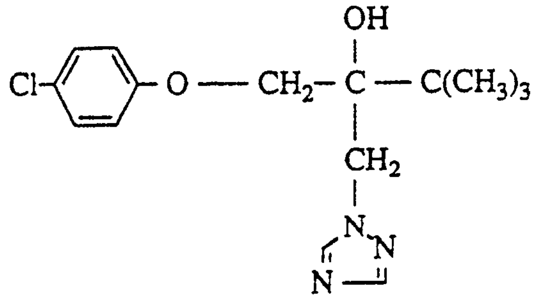

(I)

(-) Enantiomer

2. Process for the preparation of the (-)-enantiomer of 2-(4-chlorophenoxymethyl)-3,3-dimethyl-1-(1,2,4-triazol-1-yl)-2-butanol of the formula

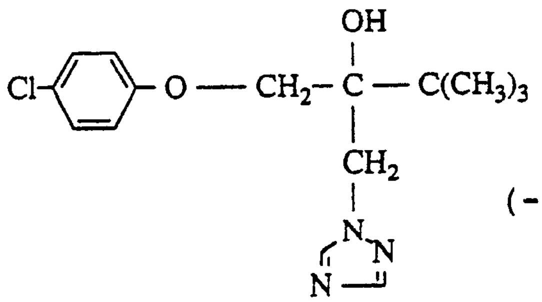

(I)

(-) Enantiomer

characterized in that in a <u>1st</u> <u>stage</u> racemic 2-(4-chlorophenoxymethyl)-2-tert.-butyl-oxirane of the formula

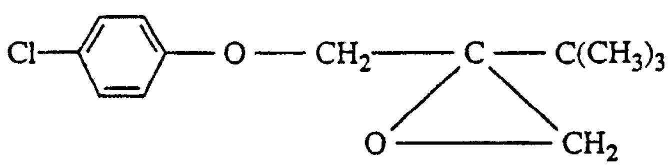

(II)

is reacted with strong optically active acids, if appropriate in the presence of a diluent, the resulting diastereomeric ester mixture is fractionated into the pure diastereomeric components and in a 2nd stage reaction is carried out with 1,2,4-triazole in the presence of a diluent and if appropriate in the presence of a base.

3. Process according to Claim 2, characterized in that camphor-10-sulphonic acid, 3-bromo-10-camphorsulphonic acid, 3-bromo-8-camphorsulphonic acid or 1,1'-binaphthyl-2,2'-diyl phosphate is used as strong optically active acid.

4. Process according to Claim 2, characterized in that the first reaction step is carried out in the temperature range from 0 to 100 °C, and the second is carried out in the temperature range from 0 to 150 °C.

5. Process according to Claim 2, characterized in that , in the first reaction step, equimolar amounts of the components are employed, and in the second reaction step 1 to 4 moles of triazole and, if appropriate, 1 to 2 moles of base are employed per mole of ester.

6. Use of the (-)-enantiomer of 2-(4-chlorophenoxymethyl)-3,3-dimethyl-1-(1,2,4-triazol-1-yl)-2-butanol of the formula (I) according to Claim 1 for the preparation of antimycotic agents.

## Revendications

1. (-)-énantiomère du 2-(4-chlorophénoxyméthyl)-3,3-diméthyl-1-(1,2,4-triazole-1-yl)-2-butanol de formule

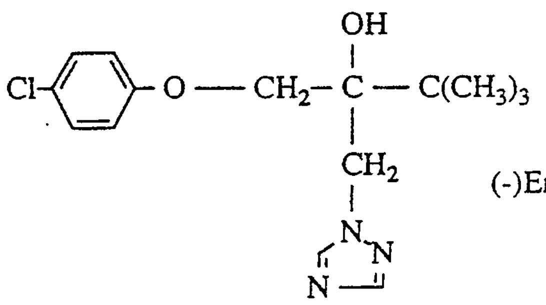

(-)Enantiomère (I)

2. Procédé de préparation due (-)-énantiomère due 2-(4-chlorophénoxyméthyl)-3,3-diméthyl-1-(1,2,4-triazole-1-yl)-2-butanol de formule

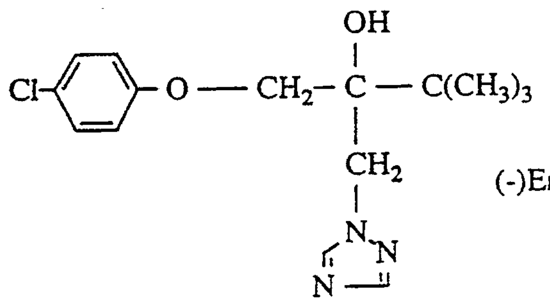

(-)Enantiomère (I)

caractérisé en ce qu'on fait réagir dans une première étape le 2-(4-chlorophénoxyméthyl)-2-tertiobutyloxiranne racémique de formule

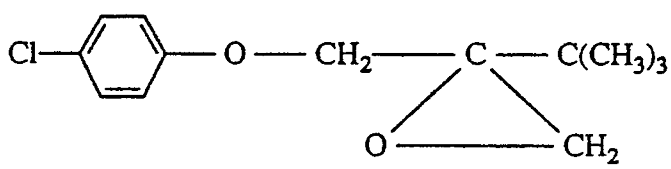

(II)

avec des acides forts, optiquement actifs, éventuellement en présence d'un diluant, on fractionne le mélange formé d'esters diastéréo-isomériques en les composants diastéréoisomériques purs, et on les fait réagir dans une seconde étape avec le 1,2,4-triazole en présence d'un diluant et, le cas échéant, en présence d'une base.

3. Procédé suivant la revendication 2, caractérisé en ce qu'on utilise comme acides forts optiquement actifs l'acide campho-10-sulfonique, l'acide 3-bromo-10-camphosulfonique, l'acide 3-bromo-8-camphosulfonique ou le phosphate de 1,1'-binaphtyl-2,2-diyle.

4. Procédé suivant la revendication 2, caractérisé en ce qu'on opère dans la plage de températures de 0 à 100 °C dans la première étape réactionnelle, dans la plage de températures de 0 à 150 °C dans la seconde étape.

5. Procédé suivant la revendication 2, caractérisé en ce qu'on utilise des quantités équimolaires des

composants dans la première étape réactionnelle, 1 à 4 moles de triazole et le cas échéant 1 à 2 moles de base par mole d'ester dans la seconde étape réactionnelle.

6. Utilisation du (-)-énantiomère du 2-(4-chlorophénoxyméthyl)-3,3-diméthyl-1-(1,2,4-triazole-1-yl)-2-butanol de formule (I) suivant la revendication 1 pour la préparation d'agents antimycosiques.